# EUROPEAN PATENT SPECIFICATION

(11) **EP 1 603 486 B1**
(45) Date of publication and mention of the grant of the patent: **20.08.2008**
(21) Application number: 04719316.4
(22) Date of filing: 10.03.2004
(51) Int. Cl.: A61F 2/00

(54) **INTERVENTIONAL CATHETERS HAVING DIFFERENTIAL CUTTING SURFACES**
INTERVENTIONSKATHETER MIT UNTERSCHIEDLICHEN SCHNEIDFLÄCHEN
CATHETERS CHIRURGICAUX POSSEDANT DES SURFACES DE COUPES DIFFERENTIELLES

(30) Priority: 10.03.2003 US 453846 P; 20.05.2003 US 442888; 20.01.2004 US 760759
(43) Date of publication of application: 14.12.2005
(73) Proprietor: Pathway Medical Technologies, Inc., Redmond, WA 98052 (US)
(72) Inventor: WULFMAN, Edward, Woodinville, Washington 98072 (US); TORRANCE, Casey, Seattle, Washington 98115 (US); NISTAL, Brent, Seattle, WA 98133 (US); YOUMANS, Scott, Bothell, Washington 98011 (US); HEFNER, Matt, Fall City, WA 98024 (US)
(74) Representative: Schneider Feldmann AG Patent- und Markenanwälte
(86) International application number: PCT/US2004/007531
(87) International publication number: WO 2004/080345

(56) References cited:
- US-A- 4 445 509
- US-A- 4 700 705
- US-A- 4 790 813
- US-A- 5 211 651
- US-A- 5 913 867
- US-A- 6 126 667
- US-B1- 6 482 217
- US-B1- 6 503 261

## Description

### Field of the Invention

The present invention relates to an interventional, catheter assembly in accordance with the preamble of claim 1 for removing material, such as obstructions and partial obstructions, from an internal lumen or cavity of a mammalian subject, such as a blood vessel, a portion of the gastrointestinal tract, dural spaces associated with the spinal cord, and the like. More particularly, the present invention relates to interventional catheters having advanceable, rotatable operating heads incorporating differential cutting surfaces.

### Background of the Invention

Removal of disease such as atherosclerotic plaque, thrombus, and other types of obstructions and partial obstructions from internal body lumens or cavities is a well-established interventional technique. Numerous interventional catheters have been conceived and developed. Most of these systems require placement of a guiding catheter and guide wire prior to introduction of the interventional catheter and placement of the interventional catheter at the target operating site. Advanceable, rotating operating heads have been used to cut and/or ablate obstructions. Many of these prior art systems incorporate aspiration systems to remove the ablated material from the site.

Despite the many and varied approaches to the material removal systems, many challenges remain in providing systems for removing material from a lumen, such as a blood vessel, safely and reliably and without causing complications. The safety and reliability of the system is manifestly critical. Recovery of debris generated during a material removal operation, or maceration of the debris to a particle size that will not produce blood vessel damage or embolic events is essential. The flexibility and size of an interventional catheter is an important feature. The system must be small enough and flexible enough to navigate through sometimes tortuous internal structures and passageways, such as blood vessels, for placement at the target interventional site. The interventional catheter must also have sufficient stiffness and integrity to operate reliably at high rotational rates while allowing for aspiration and/or infusion of fluids to the site.

In interventional catheters that employ a "cutting head," any cutter structures must be benign during navigation of the operating head to and from the interventional target site, yet effectively remove material during the operation. In addition, cutter structures must effectively remove disease or undesired material without damaging delicate neighboring tissue, such as blood vessel walls or other healthy tissue, which often surrounds and may be attached to the undesired material. Thus, it is important for cutter structures of interventional catheters to accurately and reliably differentiate between the disease or undesired material and healthy tissue.

Cutter assemblies operating according to the principles of differential cutting have been developed. Differential cutting blades exert high shear forces against relatively hard substrates to cut or ablate relatively hard, inelastic material. Softer, elastic structures, such as healthy tissue, blood vessel walls, and the like are deformed rather than cut by differential cutting blades, which reduces the shear forces and protects elastic structures from damage. Less elastic material does not deform when contacted by a differential cutting blade, and shear stresses are consequently exerted on less elastic material to cut or scrape and ablate the material without damaging elastic tissue in proximity. In this manner, fragments of diseased, undesirable material are removed by differential cutting blades, while the more elastic, healthy tissue remains undamaged.

U.S. Patent 4,445,509 describes differential cutting in the context of an atherectomy device. This patent describes a cutter assembly having a plurality of cutting flutes, each cutting flute having a blade surface operating according to the principle of differential cutting. The flute density and blade cant angle are disclosed as being key to providing effective differential cutting. Specifically, the cant angle, or angle of intersection of the cutting face and the circumference at the point of contact with the tissue is at least 90° and preferably about 110°. The face of the cutting flute also has a slight concave curvature.

Some interventional catheters use diamond grit on a cutting surface in an effort to provide highly divided, small particle size debris. Diamond grit particles, however, don't operate as differential cutters because, depending on their orientation on the cutting surface, their exposed surfaces form random cant angles, or angles of attack, to produce different cutting characteristics at different points of contact with tissue. Because not all of the diamond grit surfaces operate as differential cutting mechanisms, the diamond grit is more likely to damage elastic, healthy tissue such as blood vessel walls.

The extent and consistency of the disease or undesired material forming an obstruction are frequently not well characterized prior to intervention. Thus, although interventional catheters and cutter assemblies having different sizes and material removal properties may be provided, and may even be interchangeable on a material removal system, it is difficult to ascertain which combination of features will be most effective in any particular intervention prior to insertion of the device. Various quick-connect systems have been developed to permit removal and installation of multiple operating catheters during a single surgical intervention. This is not ideal, since interchange, withdrawal and insertion of multiple interventional catheters is time consuming, may result in increased blood loss during the intervention, and increases the risk of the operation. Having access to multiple cutter assemblies having different sizes and different material removal properties on a single interventional operating catheter is highly desirable.

In particular, it is desirable to use an interventional catheter having a small diameter during the catheter insertion and removal routine and having a cutter assembly that expands to provide a larger diameter cutter during operation when located at the target site. Numerous expandable cutter assemblies for interventional catheters have been developed, e.g. as shown in 6,565,588B1; 5,540,707; 5,192,291; 5,224,945; 5,766,192; 5,158,564; 4,895,560; 5,308,354; 5,030,201; 5,217,474; 5,140,425; and 4,966,604.

From US-A-4700 705 an interventional catheter assembly is known, comprising
a) a rotatable drive shaft in operable communication with a drive system; and
b) a cutter assembly comprising a fixed differential cutter assembly having a plurality of fixed differential cutting surfaces in a radially symmetrical arrangement with respect to a central longitudinal axis of the cutter assembly, wherein the differential cutting surfaces have a cant angle of less than 90°, the cant angle being an angle of intersection of the cutting surface and a line tangent to the circumference of the cutter assembly.

Furthermore US-A 6126 667 discloses an interventional catheter assembly with a cutter head with a surface with diamond grit wherein the cutter head has an inner cutter head member and an outer cutter head member whereby the inner cutter head member and the outer cutter head member are connected by a bearing allowing to articulate in one plane the outer part relatively to the inner part of said cutter assembly about a single axis formed by a post on the inner part which rests in a recess in the inner surface of the outer part.

### Summary of Invention

Interventional catheters of the present invention incorporate a material removal component, referred to herein as a "cutter" or "cutter assembly" or "operating head" at their distal ends. The cutter assembly is operably connected to a rotatable and axially translatable drive shaft and catheter system, drive system, and control systems. The "cutter assembly" comprises one or more distally located cutting or abrading surface(s), or blades, that operates according to the principles of differential cutting. The cutting surfaces or blades are preferably substantially rigid and multiple blades or cutting surfaces are preferably radially symmetrical. The differential cutting surfaces or blade(s) have one or more beveled edges having a cant angle, or angle of attack, of less than 90° to provide effective differential cutting.

As used herein in the description of various components, "proximal" refers to a direction toward the system controls and the operator along the path of the drive shaft and catheter system, and "distal" refers to the direction away from the system controls and the operator along the path of the drive shaft and catheter system toward or beyond a terminal end of the cutter assembly. In general, interventional catheters of the present invention comprise a cutter assembly comprising at least one differential cutting surface positioned at or near the distal end of the interventional catheter system.

Although the "cutting" surfaces or blades of interventional catheters of the present invention may be sharp and may actually "cut" material at the target site, the term "cut" or "cutting," as used herein, refers to cutting, scraping, ablating, macerating and otherwise breaking down undesired material into removable particles or smaller, removable units of material. "Cutters," "cutter assemblies," "cutting surfaces" and "blades" likewise refer to structures for cutting, scraping, ablating, macerating and otherwise breaking down material into smaller pieces.

Cutter assemblies of the present invention comprise differential cutting surfaces and generally comprise a plurality of differential cutting blades and an expandable cutter assembly thai is navigable to the intervention site in a smaller diameter condition, then adjusted to a larger diameter condition at the target site during operation, and finally withdrawn from the intervention site in a smaller diameter condition.

The drive shaft that conveys rotation and torque from a drive system to the cutter assembly must be small enough and flexible enough to be navigated through small and tortuous passageways during navigation of the cutter assembly to the target removal site, and must have sufficient mechanical integrity to transfer high rotational and torque loads and operate in a high vacuum, aspirate withdrawal environment. Multi-filar helical coils are used as drive shafts in many types of interventional catheters having a rotatable operating head. Several, preferred drive shaft embodiments are described below.

Interventional catheters of the present invention preferably include an aspiration system for removal of debris from the intervention site, generally via aspiration through one or more material removal ports provided in the cutter assembly or another component in proximity to the cutter assembly. Debris generated during a material removal operation is entrained in fluids (e.g. blood), and the aspirate fluid containing debris is removed by aspiration through the material removal ports and withdrawn through a sealed lumen of the interventional catheter. The sealed lumen is connectable to a vacuum source and aspirate collection system. The material removal ports may be disposed between blade surfaces of the cutter assembly and preferably have a large surface area.

Liquid infusion may be provided in proximity to the cutter assembly in addition to or alternatively to aspiration. Infusion of liquids may be used to provide additional liquid volume for removal of debris, or to deliver lubricating fluids, treatment agents, contrast agents, and the like. Infusion of fluids in proximity to the area of a material removal operation may be desirable because it tends to reduce the viscosity of the materials being removed, thus facilitating removal through relatively small diameter lumens. Infusion of liquids also desirably tends to reduce the volume of blood removed during a material removal operation. According to one embodiment, a sealed lumen formed between the cutter assembly drive shaft and a catheter may alternatively and selectively be used as aspirate removal system and an infusion system. The sealed lumen may thus be selectively connectable to a vacuum source and aspirate collection system for aspiration, and an infusion source for infusion of liquids. Ports in or in proximity to the cutter assembly may be thus be employed, selectively, as aspiration and infusion ports.

Interventional catheters of the present invention also incorporate an articulating bearing that improves the flexibility of the system and facilitates navigation of the cutter assembly through tortuous passageways during guidance to and from the intervention site. The bearing system couples cutter assemblies and operating heads of the present invention to the drive shaft so that torque and rotation are conveyed from the drive shaft to the cutter assembly. The bearing system also couples the cutter assembly or operating head to a static catheter system, or sheath, that provides a sealed lumen and generally does not rotate during operation of the interventional catheter. The bearing system provides enhanced flexibility at the connection that joins the operating head, drive shaft and catheter, and also provides a channel for aspiration and/or infusion of fluids.

### Brief Description of the Drawings

Figure 1 shows exemplary components of an interventional catheter assembly of the present invention having an operating head comprising differential cutting surfaces and an articulating bearing.
Fig, 2 shows a prior art differential cutter assembly described in U.S. Patent 4,445,509, wherein the differential cutter blades have cant angles of greater than 90°.
Figure 3A shows an enlarged side view of an exemplary fixed blade cutter assembly having a plurality of differential cutting surfaces and aspiration ports.
Figure 3B shows an enlarged perspective view of the fixed blade differential cutter assembly of Figure 3A.
Figure 3C shows an enlarged cross-sectional view of the fixed blade differential cutter assembly of Figures 3A and 3B illustrating differential cutting surfaces having cant angles of less than 90°.
Figures 4A and 4B show schematic diagrams illustrating the effect on a relatively elastic surface of a differential cutting surface having a 90° cant angle (Fig. 4A) as used in prior art devices and a differential cutting surface having a 60° cant angle (Fig. 4B) as used in cutter assemblies.
Figures 5A and 5B illustrate schematic cross-sectional views of differential cutter assemblies having three differential cutting surfaces (Fig. 5A) and six differential cutting surfaces (Fig. 5B).
Figures 6A and 6B illustrate another embodiment of a fixed diameter cutter assembly having a plurality of differential cutting surfaces. Fig. 6A shows a schematic isometric view of the cutter assembly and Fig. 6B shows a schematic distal end view of the cutter assembly.
Figures 7A and 7B illustrate schematic distal and proximal end views, respectively, of another embodiment of a fixed blade cutter assembly having a plurality of differential cutting surfaces.
Figures 8A and 8B illustrate additional types of cutter blades incorporating differential cutting surfaces,
Figures 9A and 9B show enlarged schematic diagrams of a composite cutter assembly of the present invention comprising two types of differential cutter surfaces in a dual cutter, with the proximal end of the cutter assembly mounted to an articulating bearing. Figure 9A shows the differential cutter and bearing assembly in an assembled condition, while Figure 9B shows the differential cutter and bearing assembly in an exploded, unassembled condition.

### Detailed Description of Preferred Embodiments

Exemplary interventional catheters and material removal systems, components and subassemblies suitable for use in connection with components of the present invention are disclosed and described in the publications incorporated herein by reference, including U.S. Patent 6,565,588B1 and PCT Patent Publication WO 01/76680. The cutter assemblies and bearing assemblies described herein may be used with interventional catheters having a variety of drive, control and other systems The cutter assembly is provided at or near a distal end of the interventional catheter and is guided to and from a desired material removal site through internal passages, such as blood vessels, as is well known in the art. At the target removal site, the cutter assembly is actuated to cut, grind or ablate, or otherwise separate and break down undesired occlusive material. In many embodiments, the occlusive material is removed the site by means of an aspiration system or another debris removal system.

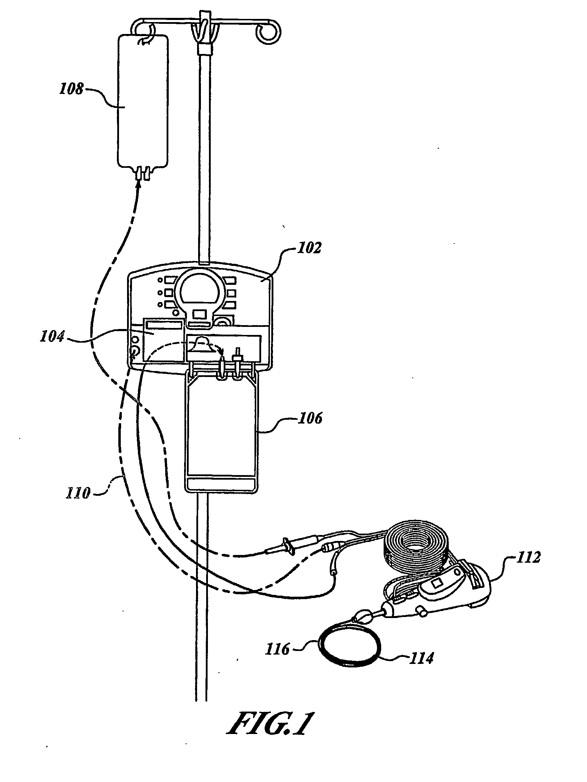

Figure 1 illustrates an exemplary interventional catheter and operating system of the present invention. In the interventional catheter and control system shown in Fig. 1, console unit 102 is provided for housing certain power, control and display functions. Console 102 may have adjustable controls that permit the operator to adjust operating parameters of the interventional catheter. Control features may include a system on/off actuator, selectable torque buttons to adjust cutter assembly rotational torque, selectable rotation features providing fixed and/or variable rotational speeds during actuation of the cutter assembly, a display of the real-time rotational speed of the cutter assembly during actuation, a timer control and/or actuation time counter to display the length of time of actual cutter operation, a motor only control to activate or shut off a rotational drive system without affecting aspiration and/or infusion, an aspiration and/or infusion only control to activate or shut off an aspiration and/or infusion function without affecting the rotational drive, and the like.

In the embodiment of Fig. 1, aspiration or vacuum motor 104 is mounted on or in console 102, as is a liquid/debris collection receptacle 106 and a liquid supply 108. Power connector 110 provides power to other interventional catheter components. Suitable electrical and liquid flow conduits are provided for operable communication between console 102 and catheter control module 112. Control module 112, in this embodiment, houses the motor drive for rotating a cutter assembly of the interventional catheter and also incorporates certain connectors and systems for communicating torque and rotation to a drive shaft having an operating head, such as a rotatable cutter assembly, mounted at or near its distal end, and for facilitating liquid infusion to and/or liquid and debris removal from the operating head. Control module 112 also incorporates systems facilitating axial translation of the interventional catheter, indicated generally at 114, and an operating head 116, such as a rotatable, advanceable cutter assembly having at least one differential cutting surface, to and from an internal target material removal site. An articulating bearing, described herein, is incorporated in proximity to the operating head to provide greater flexibility at the distal portion of the interventional catheter.

In the interventional catheter system illustrated in Fig. 1, certain power, control and display functions are provided separately in console 102 and catheter control module 112. It will he recognized that these power, control and display functions may be provided in a single unit, or may be separated in a plurality of control units. Likewise, it will be recognized that the cutter assemblies and differential cutting surfaces of the present invention may be incorporated in interventional catheters having different types of power, display and control systems, and providing different functions. Differential cutting surfaces of the present invention may be incorporated in any type of interventional catheter that may be employed for removal of undesired material located within a human or animal body.

The operating head of the interventional catheter of the present invention may comprise any of a variety of cutting devices or assemblies having one or more stiff and/or sharp cutting surface(s) for cutting, fragmentizing, pulverizing, ablating, scraping, grinding or otherwise reducing the size of and/or separating undesired material from healthy tissue, such as the walls of a blood vessel, in proximity to the target removal site. For example, the cutting surfaces may include one or a combination of blade(s), spring(s), metallic or ceramic or composite surfaces having at least one surface that operates according to the principle of differential cutting.

Some exemplary materials of construction for the cutting surface(s) of the cutter assembly include metals, metal alloys, ceramics and cermet materials, such as but not limited to, various types of stainless steels, such as series 300 and/or 400, vanadium steel, nickel-titanium, titanium, titanium-containing metals and oxide ceramics. Metallic materials such as stainless steels may be hardened using well-known techniques. In general, cutter surfaces are constructed from hard materials and may be treated to impart even greater hardness to the cutter surfaces. Cutter surfaces constructed from a material that is harder than the materials used to construct stents are generally provided. The cutter assembly, or sub-components thereof, such as the cutting surfaces, may be coated with a radio-apaque material such as gold, platinum, inks and the like, to render the expandable cutter assembly radioscopically visible and to assist a medical professional in guiding and positioning the cutter assembly relative to an occlusion.

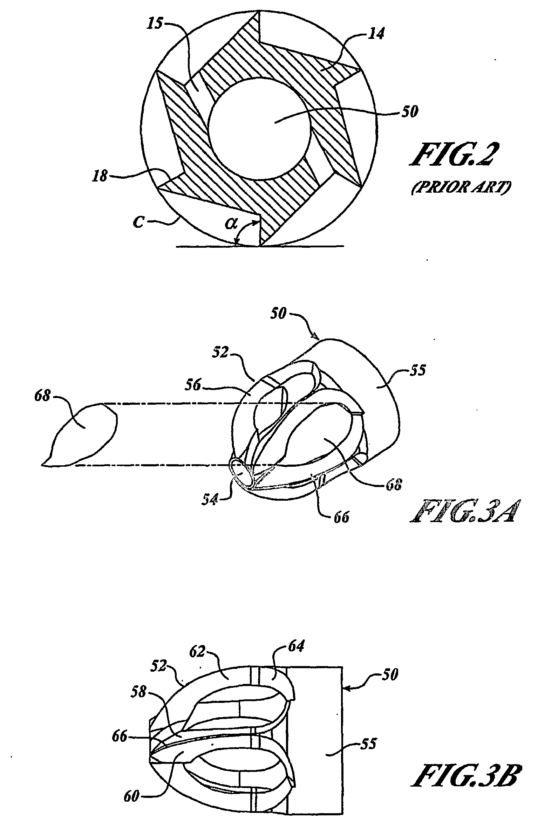

Fig. 2 illustrates an enlarged cross-sectional view of prior art differential cutter surfaces according to U.S. Patent 4,445,509 (hereinafter referred to as " '509 patent"). A plurality of cutting flutes 14 arc provided with corresponding cutting faces 18. Fluid ports 15 are provided as passageways having a rearward or longitudinal component and are intended for withdrawal of fluids and debris from the intervention site to cavity 40. The cutter assembly, as illustrated, rotates in a clockwise direction during a material removal operation. The cant angle, which is illustrated as angle α, is the of intersection of cutting face 18 and the circumference C, or a line tangent to circumference C of the cutter assembly. According to the '509 patent, cant angles of about 110°, and cant angles deviating from 110° by about 20° provide acceptable differential cutting properties.

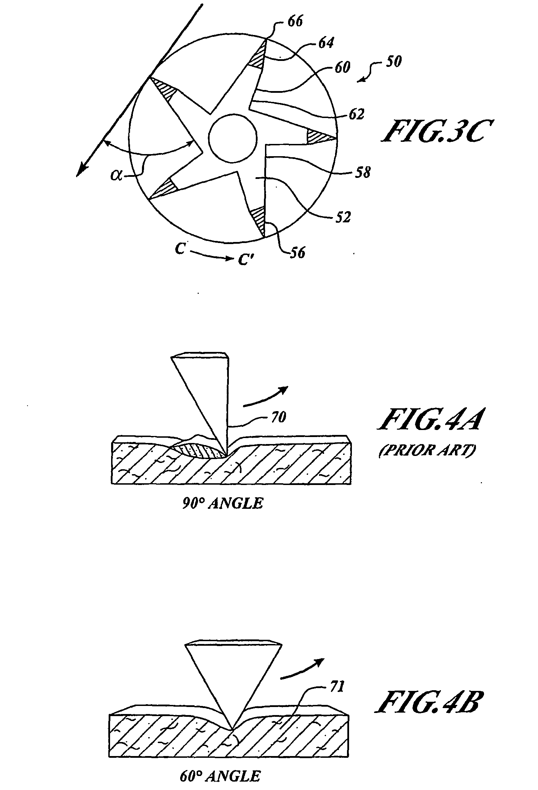

Figs. 3A-3C illustrate fixed blade cutter assemblies having a plurality of differential cutting surfaces. Cutter assembly 50 comprises a plurality of cutter blades 52 arranged in a radially symmetrical arrangement with respect to a central longitudinal axis of the cutter assembly. Each of the cutter blades 52 is joined at a distal end to form a distal bore 54 which seizes as a rotating bearing for a guidewire when a guidewire is used in a material removal operation. Cutter blades 52 may alternatively terminate at their distal ends in a blunt or pointed structure without forming a distal bore in cutter assemblies that are employed without guidance over a guidewire. Cutter blades 52 terminate at their proximal ends in a proximal ring-like collar 55 that is mounted to a drive shaft, a catheter system, or an intermediate bearing structure providing operable connections between the cutter assembly, the drive shaft, and the catheter system.

Ports 68 formed by the boundaries of neighboring pairs of cutter blades 52 and the distal and proximal rings are large and generally triangular in configuration, having curved sides. Ports 68 provide access to an internal space and aspiration lumen for removal of fluids and debris during a material removal operation. Because cutter blades incorporating differential cutting surfaces of the present invention can be provided as thin blades, the port size can be maximized. The total cross-sectional area of the ports, compared to the total cross-sectiono,l of the inside surfaces of the cutter blades in cutter assemblies of the present invention is generally at least 1.5:1, preferably at least 2:1, more preferably at least 2.5:1; and more preferably yet at least 3:1.

Each of the cutter blades 52 has a differential cutter surface 56 that contacts material to be removed when cutter assembly 50 is rotated during a material removal operation. In the cutter assembly embodiment shown in Figs. 3A-3C, cutter assembly 50 is configured to rotate in a counterclockwise direction. Differential cutter surfaces of the present invention have a cant angle α, which is the angle of intersection of cutter surface 56 and a line tangent to the circumference of the cutter assembly, of less than 90°,

Differential cutter surfaces may be optimized for use in different types of cutting environments, for different types of materials being removed, and for different applications, by providing different cant angles, providing that the cant angle for differential cutting is less than 90°. Because differential cutter surfaces having cant angles of less than 90° provide more effective differential cutting, fewer cutter surfaces may be required for material removal operations. In general, in cutter assemblies having fewer blades, the blades have a thin cross-sectional dimension, allowing for larger aspiration ports and greater aspiration effcicncy. Cutter assembles are benign to healthy, elastic tissue while providing effective removal of less elastic, disease tissue and providing high aspiration rates for removal of debris.

Cutter surfaces 56 may be provided on a leading blade surface 58 that is a planar surface. Trailing blade surfaces 60 may comprise a compound surface composed of two or more adjoining surfaces 62 and 64 provided at angles to one another. In the embodiment shown in Figs. 3A-3C, leading blade surfaces 58 are longer than trailing blade surfaces 60, and leading blade surfaces 58 intersect trailing blade surfaces 60 at an inner region at a substantially right angle. The outer terminal edges 66 of blades 52 formed at the intersection of cutter surfaces 56 and trailing blade surfaces 60 preferably form an acute angle to one another and are preferably sharpened. In many embodiments of cutter assemblies of the present invention, neither the leading nor the trailing blade surfaces are aligned radially with respect to the longitudinal axis of the cutter assembly.

Figure 4 illustrates, schematically, the cutting action of a blade 70 having a 90° cant angle rotating in a counterclockwise direction (Fig. 4A) and the cutting action of a blade 71 having a 60° cant angle rotating in a counterclockwise direction (Fig. 4B). Differential cutting blade 71 having a 60° cant angle, as shown in Fig. 4B, is gentle and benign when it contacts a resilient surface, yet it effectively cuts and abrades less resilient materials to provide effective removal of disease material such as plaque, calcified material and thrombus. Cutting blade 70 having a 90° cant angle, as shown in Fig. 4A, can be quite damaging to resilient surfaces. The laboratory tests described in Example 2, below, provide data confirming the improved properties of cutting blades having a 60° cant angle compared to cutting blades having a 90° cant angle.

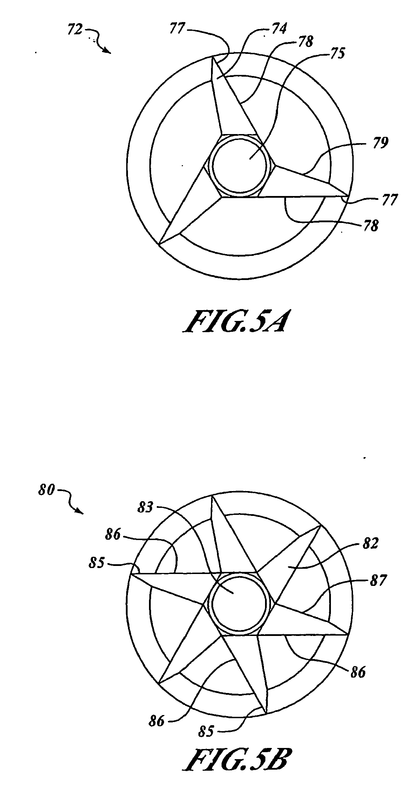

Figures 5A and 5B illustrate cutter assemblies having different numbers and arrangements of differential cutting surfaces. Any number of cutter blades may be employed in cutter assemblies and interventional catheters Cutter assemblies having three, five, six and seven cutter blades that arc radially symmetrical arc especial preferred for many applications.

Fig. 5A shows a cutter assembly 72 having three radially symmetrical cutter blades 74 that provide differential cutting when cutter assembly 72 is rotated in a clockwise direction. Cutter blades 74 terminate at a distal end in distal bore 75 and terminate at a proximal end in proximal collar. Cutter surfaces 77 have a cant angle α of less the 90°. Cutter surfaces 77 are provided on a leading blade surface 78 that is a planar surface. Trailing blade surfaces 79 have a tapered configuration. In the embodiment shown in Fig. 5A, leading blade surfaces 78 are longer than trailing blade surfaces 79, and leading blade surfaces 78 intersect trailing blade surfaces 79 at an inner region at an obtuse angle.

Fig. 5B shows an embodiment of cutter assembly 80 having six radially symmetrical cutter blades 82 that provide differential cutting when cutter assembly 80 is rotated in a clockwise direction. Cutter blades 82 terminate at a distal end in distal bore 83 and terminate at a proximal end in proximal collar. Cutter surfaces 85 have a cant angle α of less the 90°. Cutter surfaces 85 are provided on a leading blade surface 86 that is a planar surface. Trailing blade surfaces 87 have a tapered or compound configuration. In the embodiment shown in Fig. 5B, leading blade surfaces 86 intersect trailing blade surfaces 87 at an inner region at an acute angle.

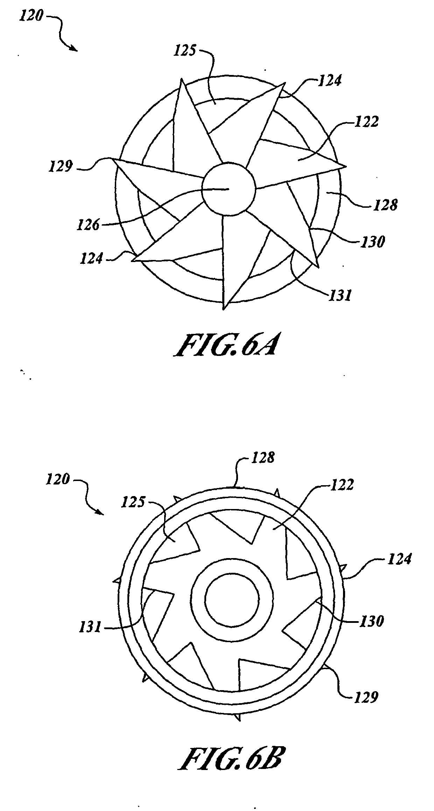

Figures 6A and 6B illustrate yet another cutter assembly 120 having a plurality of cutting blades 1.22, each cutting blade being provided with a differential cutting surface 124. Cutter assembly 120 has seven cutter blades provided in a radially symmetrical, arrangement with ports 125 provided between each neighboring pair of cutter blades. Ports 125 are large and have a generally ovoid configuration with curved walls. Cutter assembly 120 is rotatable in a clockwise direction to provide differential cutting.

Cutter blades 122 terminate at a distal end in distal bore 126 and terminate at a proximal end in proximal collar 128. Cutter surfaces 124 have a cant angle α of less the 90°. Cutter surfaces 124 are provided on a leading blade surface 131 that is a planar surface. Trailing blade surfaces 130 have a tapered or compound configuration. In the embodiment shown in Figs. 6A and 6B, edges 129 of cutter surfaces 124 at their largest diameter point extend beyond the diameter of proximal collar 128. Edges 129 have a curved profile along their length between distal bore 126 and proximal collar 128 that extends to a larger diameter than proximal collar 128 and then tapers to join proximal collar 128. This cutter blade profile permits effective removal of material to form a passageway through obstructions that is larger in diameter than the proximal collar of the cutter assembly and promotes side cutting. This cutter blade profile may be particularly advantageous for use in interventional catheter systems in which the cutter assembly is guided by remote steering technologies rather than being advanced on a guidewire.

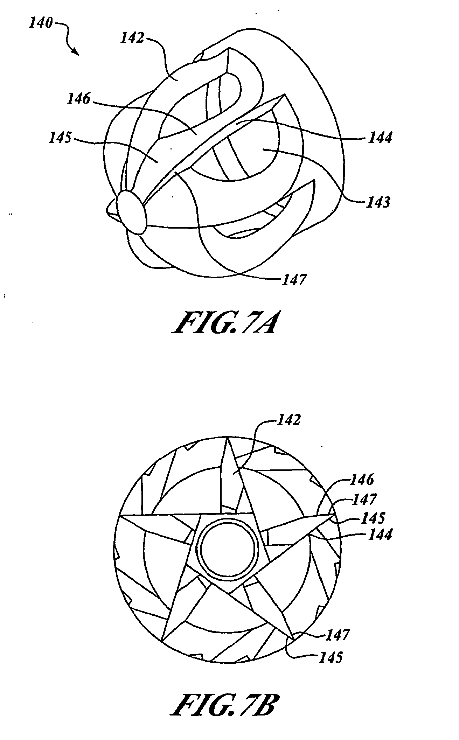

Figures 7A and 7B illustrate yet another cutter assembly 140 having a plurality of differential cutting blades 142 and aspiration ports 143. In this embodiment, both leading blade surfaces 144 and trailing surfaces 146 provide differential cutting surfaces and both leading and trailing blade surfaces may be compound surfaces. Leading blade surfaces 144 form differential cutting surfaces 145, while trailing blade surfaces 146 form differential cutting surfaces 147. Differential cutting surfaces 145 and 147 all have cant angles of less than 90° to provide effective and safe differential cutting properties.

Differential cutting surfaces 145 and 147 operate when cutter assembly 140 is rotated in clockwise and counterclockwise directions, respectively. Providing differential cutting surfaces on both the leading and trailing surfaces of cutting blades dramatically increases the versatility of the cutter assembly when it is used in conjunction with a bi-directional motor drive. In preferred embodiments, differential cutting surfaces 145 and 147 have different cant angles to provide different differential cutting properties. Differential cutting surfaces 145 may have a cant angle of less than 90° and more than about 70°, while differential cutting surfaces 147 may have a cant angle of less than 70° and more than 30°. The operator may select the direction of drive shaft and cutter assembly rotation to employ the differential cutter blades having the differential cutting properties most suitable for removal of particular material during a material removal process. Various combinations of differential cutter blades and differential cutting surfaces having various cant angles may be used in differential cutting blades having dual differential cutting surfaces.

The fixed blade cutter assemblies described above have multiple cutter blades and multiple differential cutter surfaces. The cutter assemblies and cutter blades may be provided in various configurations, presenting various external profiles, and having various port surface area to internal blade surface area ratios. In general, the plurality of differential cutting surfaces provided in a cutter assembly having a single differential cutting surface on each blade each have the same cant angle. In alternative embodiments, different cant angles may be provided on single differential cutting surfaces on individual cutting blades forming a cutter assembly. Thus, for example, a fixed blade cutter assembly having six radially symmetrical cutter blades may be provided having three cutter blades with differential cutting surfaces having a first cant angle less than 90° and three cutter blades with differential cutting surfaces having a second cant angle less than 90° and different from the first cant angle. The cutter blades having differential cutting surfaces with different cant angles are preferably provided in an alternating arrangement.

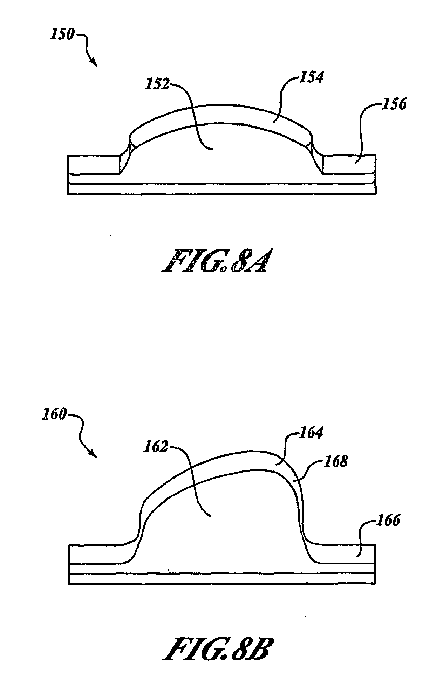

Figures 8A and 8B illustrate yet additional types of differential cutting blades 150 and 160 that may be used, for example, as pivoting differential cutting blades in an expandable type of cutting assembly, which is described in more detail below. Differential cutting blade 150 comprises a generally planar surface 152 having a differential cutting surface 154 at a peripheral edge. Planar surface 152 may be mounted on or integral with mounting tabs or rods 156 for mounting blades 150 to a cutter assembly structure. Differential cutting blade 160 comprises a generally planar surface 162 having a differential cutting surface 164 at a peripheral edge and a mounting rod 166 for mounting blades 160 to a cutter assembly structure. Differential cutting surfaces 154 and 164 have cant angles, as defined above, of less than 90°.

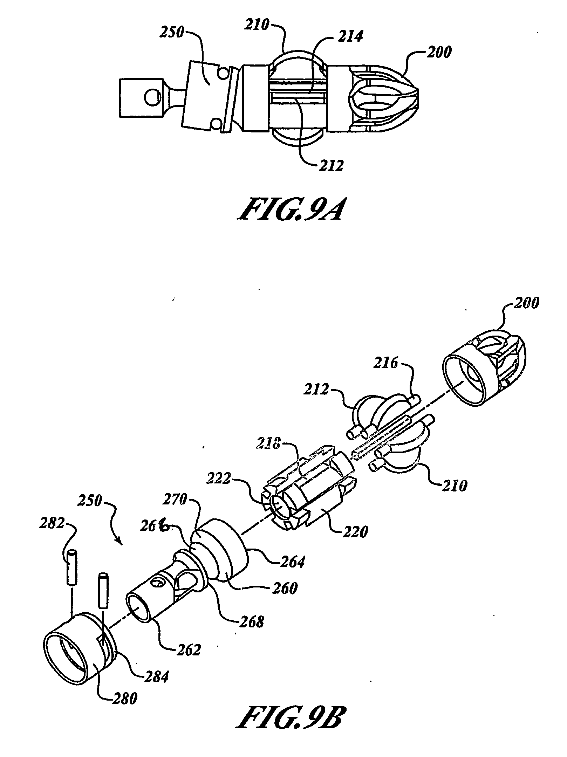

Figures 9A and 9B illustrate dual differential cutter assemblies having a fixed differential cutter assembly 200, an adjustable differential cutter assembly 210 shown in an expanded diameter condition in which the blades are in a radial configuration, and a bearing 250 communicating between the cutter assemblies, the drive shaft, and the catheter assembly. Fixed cutter assembly 200 is preferably provided at a distal end of the assembly, with adjustable cutter assembly 210 mounted proximally to the fixed cutter assembly. Suitable fixed cutter assemblies have been described above, In the embodiment shown in Figs 9A and 9B, differential cutting blades provided on the fixed cutter assembly and on the differential cutter assembly have approximately the same length. In alternative embodiments, differential cutting blades on either the fixed or adjustable blade cutter may have different lengths.

Adjustable diameter differential cutter assembly 210 comprises a plurality of generally planar cutter blades 212, each having at least one differential cutting surface 214 extending substantially along a peripheral edge. Differential cutting surfaces 214 have cant angles of less than 90°, as described in detail above. In general, from three to seven or more pivotable differential cutting blades may be provided on adjustable cutter assembly 210. The cant angles of differential cutting surfaces 214 may be the same as or different from cant angles of differential cutting surfaces provided on fixed differential cutter assembly 200. In a preferred embodiment, the cant angles of differential cutting surfaces 214 are less than the cant angles of differential cutting surfaces provided on fixed differential cutter assembly 200.

Mounting rods 216 of cutter blades 212 are pivotably mounted in mating slots 218 of adjustable cutter support 220 having a central passageway 222 communicating with a central bore of the distal cutter and a central bore of bearing assembly 250. Central passageway 222 generally forms part of an aspiration and/or infusion lumen. Cutter support 220 is configured to support cutter blades 212 in a tangential position, in which they lie flat against a support face to present a smaller diameter profile and in a radial position, in which they extend radially to present a larger diameter, differential cutting profile. The cutter blades may be adjusted between the tangential and radial orientations by changing the direction of rotation of the drive system, or using alternative mechanisms. Aspiration and/or infusion ports may be provided in cutter support 220.

Bearing assembly 250 provides operable coupling of an operating head, such as dual cutter assemblies 200 and 210, directly or indirectly to a drive shaft that transfers torque and rotation to the operating head. The bearing assembly is also operably coupled, directly or indirectly, to a static (non-rotating) catheter system providing a sealed lumen for aspiration and/or infusion of liquids. Bearing system 250 articulates to improve the overall flexibility and guidability of the operating head as it is navigated to and from the material removal site and provides an internal channel for aspiration and/or infusion of liquids through the bearing system.

Bearing system 250 comprises an internal shaft 260 coupled, directly or indirectly, to the drive shaft at a proximal end 262 and coupled, directly or indirectly, to the operating head at a distal end 264. Bearing system 250 additionally comprises a cylindrical static member 280 provided as an outer sleeve that is fixedly coupled at or near a proximal end to a distal end of the catheter system to provide a sealed internal lumen and catheter system. Internal shaft 260 and static member 280 are operably coupled by one or more connecting structures, such as rods 282 retained in slots 284 formed in static member 280.

Static member 280 is mounted on a peripheral surface of internal shaft 260 so that rods 282 are retained in slots 284 and ride in a curved annular seat 266 formed on internal shaft 260. Curved annular seat 266 has a variable diameter, with a central smaller diameter section flaring in both distal and proximal directions to larger diameter distal and proximal boundaries 268 and 270, respectively. Rods 282 ride in the curved annular seat to provide limited pivoting of internal shaft 260 and the operating head with respect to static member 280 and the catheter assembly. The profile and depth of the curved annular seat may be adjusted and configured to provide a desired degree of articulation. This bearing system thus provides limited articulation of the operating head with respect to the catheter assembly and facilitates both navigation of the operating head to and from the target material removal site, and operation of the operating head at the target material site to ablate occlusive material.

Drive shafts for use in interventional catheters of the present invention generally comprise helical coil or braided shafts may comprise composite drive shafts having variable flexibility and/or torque carrying capability at different sections along their length. In one embodiment, a helical coil drive shaft is provided having a less flexibility and higher torque carrying capability at a proximal portion and greater flexibility and less torque carrying capability at a distal portion. The proximal portion of the drive shaft may comprise a helical coil shaft having more files than the helical coil shaft forming the distal portion of the drive shaft. In one embodiment, a proximal drive shaft section comprises a tri- or quad-filar helical coil, while a distal drive shaft section comprises a bi- or tri-filar helical coil, respectively. In a drive shaft having a braided construction, a proximal drive shaft section comprises more strands than a distal drive shaft section.

In another embodiment, a proximal drive shaft section comprises a multi-filar helical coil in which the adjacent threads are closely spaced, while a distal drive shaft section comprises a single or multi-filar helical coil in which the adjacent threads are spaced from one another bv a space corresponding to at least the diameter of the thread. The spaces may be greater, depending on the flexibility and torque-carrying requirements of the distal drive shaft section. A preferred drive shaft may comprise a distal section having a helical coil with spaced apart threads over which a sealing member is installed. The sealing member is preferably no thicker than the diameter of a drive shaft thread and is constructed from durable, flexible plastic materials such as polyfluorotetraethylene (PFTE). A heat shrinkable PFTE tube may be installed and shrunk to conform generally to the configuration of the distal drive shaft spaced apart threads to provide a sealed distal drive shaft lumen, and to improve the lubricity of the distal drive shaft section as it is navigated to and from the target material removal site. The outer sealed layer may extend a distance of from about 2 cm to about 40 cm from the distal end of the drive shaft, more preferably from about 5 cm to about 15 cm from the distal end of the drive shaft.

## Claims

1. An interventional catheter assembly comprising:
a. a rotatable drive shaft in operable communication with a drive system; and
b. a cutter assembly (50, 72, 80, 116, 120, 140) comprising a fixed differential cutter assembly (50, 72, 80, 120, 140, 200) having a plurality of fixed differential cutting surfaces (52, 74, 77, 82, 85, 124, 145, 147) in a radially symmetrical arrangement with respect to a central longitudinal axis of the cutter assembly (50, 72, 80, 116, 120, 140), wherein the differential cutting surfaces (52, 74, 77, 82, 85, 124, 145, 147) have a cant angle of less than 90°, the cant angle being an angle of intersection of the cutting surface (52, 74, 77, 82, 85, 124, 145, 147) and a line tangent to the circumference of the cutter assembly (50, 72, 80, 116, 120, 140); **characterised in that**
c. the cutter assembly (50, 72, 80, 116, 120, 140) additionally comprises an adjustable blade cutter assembly (210) having a plurality of adjustable blades (150, 152, 160, 162, 212), and the assembly further comprises
d. a bearing system (250) coupling the cutter assembly (50, 72, 80, 116, 120, 140) to the drive shaft and to a sealed lumen, the bearing system (250) providing limited articulation of the cutter assembly (50, 72, 80, 116, 120, 140) with respect to the axis of the drive shaft, wherein the bearing system (250) comprises an internal shaft (260) coupled to the drive shaft and a static member (280) provided as an outer sleeve, the static member (280) being fixedly coupled at or near a proximal end to a distal end of a catheter system to provide a sealed internal lumen and catheter system.

2. An interventional catheter assembly of claim 1, wherein rods (282) retained in slots (284) formed on the static member (280) ride in a curved annular seat (266) formed on the internal shaft (260) to provide limited pivoting of the internal shaft (260) with respect to the static member (280).

3. An interventional catheter assembly of claim 1 and 2, wherein the fixed differential cutting surfaces (52, 74, 77, 82, 85, 124, 145, 147) have a cant angle of less than 80°.

4. An interventional catheter assembly of any one of claims 1 and 3, wherein the external profile of the fixed blade cutter assembly (50, 72, 80, 116, 120, 140) is generally ovoid or conical from a profile view.

5. An interventional catheter assembly of any one of claims 1-4, wherein the fixed differential cutting surfaces (52, 74, 77, 82, 85, 124, 145, 147) taper along a curved line between a smaller diameter distal end and a larger diameter proximal end of the cutter assembly (50, 72, 80, 116, 120, 140).

6. An interventional catheter assembly of any of claims 1-5, additionally comprising an aspiration system including a sealed lumen (222) connectible to a vacuum source (104), and wherein the cutter assembly (50, 72, 80, 116, 120, 140) additionally comprises a plurality of ports (68, 125, 143) providing access to the sealed lumen (222).

7. An interventional catheter assembly of claim 6, wherein the ratio of a total cross-sectional area of ports (68, 125, 143) compared to a total cross-sectional area of internal cutter blade surfaces is at least 1.5:1.

8. An interventional catheter assembly of any one of claims 1-7 additionally comprising a liquid infusion system including a sealed lumen connectible to an infusion source.

9. An interventional catheter assembly of any one of claims 1-8, wherein each differential cutting surface (52, 74, 77, 82, 85, 124, 145, 147) is provided on a generally planar leading blade surface (78, 86, 131, 144).

10. An interventional catheter assembly of any one of claims 1-9, wherein each differential cutting surface (52, 74, 77, 82, 85, 124,145,147) is provided at an outer terminal edge of a cutter blade (74, 82, 122, 142), and the angle formed between the differential cutting surface (52, 74, 77, 82, 85, 124, 145, 147) and a trailing blade surface (79, 87, 130, 146) is an acute angle.

11. An interventional catheter assembly of claim 9, wherein the outer terminal edge of each cutter blade is sharpened.

12. An interventional catheter assembly any one of claims 1-11, wherein each differential cutting surface (52, 74, 77, 82, 85, 124, 145, 147) is provided on a cutter blade (74, 82, 122, 142), each cutter blade (74, 82, 122, 142) terminates at a proximal end in a proximal collar (55, 128), and each differential cutting surface (52, 74, 77, 82, 85, 124, 145, 147) has an external profile that, at its largest diameter, is larger than the diameter of the proximal collar (55, 128).

13. An interventional catheter assembly of claim 12, wherein the adjustable blade cutter assembly (210) is positioned proximal to the fixed differential cutter assembly (50, 72, 80, 116, 120, 140).

14. An interventional catheter assembly of any one of claims 12 and 13, wherein the adjustable blade cutter assembly (210) has a plurality of radially arranged cutter blades (150, 152, 160, 162, 212) pivotably adjustable between a tangential orientation and a radial orientation.

15. An interventional catheter assembly of any one of claims 12-14, wherein the adjustable blades (150, 152, 160, 162, 212) have differential cutting surfaces (154, 164, 214) and the differential cutting surfaces on the fixed blade cutter assembly (52, 74, 77, 82, 85, 124, 145, 147) and differential cutting surfaces on the adjustable blade cutter assembly (154, 164, 214) have different cant angles.

16. An interventional catheter assembly of any one of claims 1-15, wherein the drive shaft is less flexible and has higher torque carrying capacity at a proximal portion and is more flexible and has lower torque carrying capacity at a distal portion.

17. An interventional catheter assembly of claim 16, wherein the drive shaft is a helical coil and, at a distal portion of the drive shaft, threads comprising the helical coil are spaced apart from one another by a space corresponding to at least the diameter of a thread.

18. An interventional catheter assembly of any one of claims 1-17, additionally comprising a sealing member installed over the distal portion of the drive shaft, wherein the sealing member closely contacts but is not bonded to the distal portion of the drive shaft.

19. An interventional catheter assembly of claim 18, wherein the sealing member is formed from a durable, flexible plastic material.

20. An interventional catheter assembly of claim 19, wherein the sealing member is formed from PFTE.

21. An interventional catheter assembly of any one of claims 1-20, additionally comprising a console unit (102) in communication with the rotatable drive shaft wherein the console unit houses power, control and display functions.

22. An interventional catheter assembly of any one of claims 1-21, additionally comprising a control module (112) wherein the control module houses a drive motor in operable communication with the rotatable drive shaft.

23. An interventional catheter assembly of any one of claims 1-22, wherein the fixed differential cutting surfaces (52, 74, 77, 82, 85, 124, 145, 147) have a cant angle of less than 70°.

24. An interventional catheter assembly of any one of claims 1-23, additionally comprising a control system. (102, 112) for operating the drive shaft and the cutter assembly, wherein the control system (102, 112) comprises a selectable torque feature for adjusting the cutter assembly rotational torque.

25. An interventional catheter assembly of any one of claims 1-24, additionally comprising a control system (1.02, 1.12) for operating the drive shaft and the cutter assembly, wherein the control system (102, 112) comprises a selectable rotation feature to provide fixed and/or variable rotational speeds during actuation of the cutter assembly.

26. An interventional catheter assembly of any one of claims 1-25, additionally comprising a control system (102, 112) for operating the drive shaft and the cutter assembly, wherein the control system (102, 112) comprises a motor only control to activate or shut off the drive shaft without affecting aspiration and/or infusion.

27. An interventional catheter assembly of any one of claims 1-26, additionally comprising a control system (102, 112) for operating the drive shaft and the cutter assembly, wherein the control system (102, 112) comprises an aspiration and/or infusion only control to activate or shut off an aspiration and/or infusion function without affecting the drive shaft.

28. An interventional catheter assembly of claim 12, additionally comprising a control feature for adjusting the adjustable blade cutter assembly (2.10) between a larger diameter condition and a small diameter condition.

## Patentansprüche

1. Eingriffskatheteraufbau, welcher das Folgende umfasst:
a. eine drehbare Antriebswelle in Bedienungskommunikation mit einem Antriebssystem;
b. einen Schneidwerkaufbau (50, 72, 80, 116, 120, 140), welcher einen festen Schneidwerkaufbau (50, 72, 80, 120, 140, 200) zum differentiellen Schneiden umfasst, der mehrere feste Schneidflächen (52, 74, 77, 82, 85, 124, 145, 147) zum differentiellen Schneiden in einer radial symmetrischen Anordnung bezüglich einer zentralen Längsachse des Schneidwerkaufbaus (50, 72, 80, 116, 120, 140) aufweist, wobei die Schneidflächen (52, 74, 77, 82, 85, 124, 145, 147) zum differentiellen Schneiden einen Neigungswinkel von weniger als 90° aufweisen, wobei es sich bei dem Neigungswinkel um einen Schnittwinkel zwischen der Schneidfläche (52, 74, 77, 82, 85, 124, 145, 147) und einer Tangente des Umfangs des Schneidwerkaufbaus (50, 72, 80, 116, 120, 140) handelt:
wobei der Schneidwerkaufbau (50, 72, 80, 116, 120, 140) außerdem einen Schneidwerkaufbau mit verstellbaren Klingen (210) umfasst, welcher mehrere verstellbare Klingen (150, 152, 160, 162, 212) aufweist; und
c. ein Lagersystem (250), welches den Schneidwerkaufbau (50, 72, 80, 116, 120, 140) mit der Antriebswelle und einem versiegelten Lumen koppelt, wobei das Lagersystem (250) für eine begrenzte Gelenkverbindung des Schneidwerkaufbaus (50, 72, 80, 116, 120, 140) bezüglich der Achse der Antriebswelle sorgt, wobei das Lagersystem (250) eine innere Welle (260), welche mit der Antriebswelle verbunden ist, und ein statisches Element (280) umfasst, welches als eine äußere Hülse bereitgestellt ist, wobei das statische Element (280) an oder nahe einem proximalen Ende fest mit einem distalen Ende eines Kathetersystems verbunden ist, um ein versiegeltes inneres Lumen- und Kathetersystem bereitzustellen.

2. Eingriffskathetersystem nach Anspruch 1, wobei Stäbe (282), welche in Nuten (284) gehalten werden, die auf dem statischen Element (280) ausgebildet sind, in einer gekrümmten ringförmigen Aufnahme (266) laufen, der auf der inneren Welle (260) ausgebildet ist, um für eine begrenzte Drehbewegung der inneren Welle (260) in Bezug auf das statische Element (280) zu sorgen.

3. Eingriffskathetersystem nach Anspruch 1 und 2, wobei die festen Schneidflächen (52, 74, 77, 82, 85, 124, 145, 147) zum differentiellen Schneiden einen Neigungswinkel von weniger als 80° aufweisen.

4. Eingriffskathetersystem nach einem der Ansprüche 1 und 3, wobei das äußere Profil des Festklingen-Schneidwerkaufbaus (50, 72, 80, 116, 120, 140) im Allgemeinen oval oder konisch ist.

5. Eingriffskathetersystem nach einem der Ansprüche 1 bis 4, wobei die festen Schneidflächen (52, 74, 77, 82, 85, 124, 145, 147) zum differentiellen Schneiden entlang einer gekrümmten Linie zwischen einem distalen Ende mit kleinerem Durchmesser und einem proximalen Ende mit größerem Durchmesser des Schneidwerkaufbaus (50, 72, 80, 116, 120, 140) konisch zulaufen.

6. Eingriffskathetersystem nach einem der Ansprüche 1 bis 5, welches außerdem ein Absaugsystem umfasst, welches ein versiegeltes Lumen (222) aufweist, das an eine Vakuumquelle (104) angeschlossen werden kann, und wobei der Schneidwerkaufbau (50, 72, 80, 116, 120, 140) außerdem mehrere Anschlüsse (68, 125, 143) umfasst, die einen Zugang zu dem versiegelten Lumen (222) bereitstellen.

7. Eingriffskathetersystem nach Anspruch 6, wobei das Verhältnis einer Gesamt-Querschnittsfläche der Anschlüsse (68, 125, 143) zu einer Gesamt-Querschnittsfläche der inneren Schneidklingenflächen mindestens 1,5:1 beträgt.

8. Eingriffskathetersystem nach einem der Ansprüche 1 bis 7, welches außerdem ein Flüssigkeitsinfusionssystem umfasst, welches ein versiegeltes Lumen aufweist, das an eine Infusionsquelle angeschlossen werden kann.

9. Eingriffskathetersystem nach einem der Ansprüche 1 bis 8, wobei jede Schneidfläche (52, 74, 77, 82, 85, 124, 145, 147) zum differentiellen Schneiden auf einer im Allgemeinen ebenen voreilenden Klingenfläche (78, 86, 131, 144) bereitgestellt ist.

10. Eingriffskathetersystem nach einem der Ansprüche 1 bis 9, wobei jede Schneidfläche (52, 74, 77, 82, 85, 124, 145, 147) zum differentiellen Schneiden an einer äußeren Abschlusskante einer Schneidklinge (74, 82, 122, 142) bereitgestellt ist und der zwischen der Schneidfläche (52, 74, 77, 82, 85, 124, 145, 147) zum differentiellen Schneiden und einer nacheilenden Klingenfläche (79, 87, 130, 146) gebildete Winkel ein spitzer Winkel ist.

11. Eingriffskathetersystem nach Anspruch 9, wobei die äußere Abschlusskante jeder Schneidklinge geschärft ist.

12. Eingriffskathetersystem nach einem der Ansprüche 1 bis 11, wobei jede Schneidfläche (52, 74, 77, 82, 85, 124, 145, 147) zum differentiellen Schneiden auf einer Schneidklinge (74, 82, 122, 142) bereitgestellt ist, jede Schneidklinge (74, 82, 122, 142) an einem proximalen Ende in einer proximalen Basishalterung (55, 128) endet und jede Schneidfläche (52, 74, 77, 82, 85, 124, 145, 147) zum differentiellen Schneiden ein äußeres Profil aufweist, welches an seinem größten Durchmesser größer ist als der Durchmesser der proximalen Basisha1terung (55, 128).

13. Eingriffskathetersystem nach einem der Ansprüche 1 bis 12, wobei der Schneidwerkaufbau (210) mit verstellbaren Klingen proximal zu dem festen Schneidwerkaufbau (50, 72, 80, 116, 120, 140) zum differentiellen Schneiden angeordnet ist.

14. Eingriffskathetersystem nach einem der Ansprüche 1 bis 13, wobei der Schneidwerkaufbau mit verstellbaren Klingen (210) mehrere radial angeordnete Schneidklingen (150, 152, 160, 162, 212) aufweist, welche durch Drehen zwischen einer tangentialen Ausrichtung und einer radialen Ausrichtung verstellbar sind.

15. Eingriffskathetersystem nach einem der Ansprüche 1 bis 14, wobei die verstellbaren Klingen (150, 152, 160, 162, 212) Schneidflächen (154, 164, 214) zum differentiellen Schneiden aufweisen und die Schneidflächen zum differentiellen Schneiden auf dem Festklingen-Schneidwerkaufbau (52, 74, 77, 82, 85, 124, 145, 147) und die Schneidflächen zum differentiellen Schneiden auf dem Schneidwerkaufbau mit verstellbaren Klingen (154, 164, 214) unterschiedliche Neigungswinkel aufweisen.

16. Eingriffskathetersystem nach einem der Ansprüche 1 bis 15, wobei die Antriebswelle in einem proximalen Bereich weniger flexibel ist und eine höhere Drehmomentkapazität aufweist und in einem distalen Bereich flexibler ist und eine geringere Drehmomentkapazität aufweist.

17. Eingriffskathetersystem nach Anspruch 16, wobei es sich bei der Antriebswelle um eine spiralförmige Feder handelt und in einem distalen Bereich der Antriebswelle Windungen der spiralförmigen Feder einen Abstand voneinander aufweisen, welcher mindestens dem Durchmesser einer Windung entspricht.

18. Eingriffskathetersystem nach einem der Ansprüche 1 bis 17, welches außerdem ein Dichtungselement umfasst, das über dem distalen Bereich der Antriebswelle installiert ist, wobei das Dichtungselement in engem Kontakt zu dem distalen Bereich der Antriebswelle steht, aber nicht mit diesem verbunden ist.

19. Eingriffskathetersystem nach Anspruch 18, wobei das Dichtungselement aus einem haltbaren flexiblen Kunststoffmaterial gebildet ist.

20. Eingriffskathetersystem nach Anspruch 19, wobei das Dichtungselement aus PFTE gebildet ist.

21. Eingriffskathetersystem nach einem der Ansprüche 1 bis 20, welches außerdem eine Konsoleneinheit (102) in Kommunikation mit der drehbaren Antriebswelle umfasst, wobei die Konsoleneinheit Stromversorgungs-, Steuerungs- und Anzeigefunktionen beherbergt.

22. Eingriffskathetersystem nach einem der Ansprüche 1 bis 21, welches außerdem ein Steuermodul (112) umfasst, wobei das Steuermodul einen Antriebsmotor in Bedienungskommunikation mit der drehbaren Antriebswelle beherbergt.

23. Eingriffskathetersystem nach einem der Ansprüche 1 bis 22, wobei die festen Schneidflächen (52, 74, 77, 82, 85, 124, 145, 147) zum differentiellen Schneiden einen Neigungswinkel von weniger als 70° aufweisen.

24. Eingriffskathetersystem nach einem der Ansprüche 1 bis 23, welches außerdem ein Steuerungssystem (102, 112) zum Bedienen der Antriebswelle und des Schneidwerkaufbaus umfasst, wobei das Steuerungssystem (102, 112) ein auswählbares Drehmomentmerkmal zum Einstellen des Drehmoments des Schneidwerkaufbaus umfasst.

25. Eingriffskathetersystem nach einem der Ansprüche 1 bis 24, welches außerdem ein Steuerungssystem (102, 112) zum Bedienen der Antriebswelle und des Schneidwerkaufbaus umfasst, wobei das Steuerungssystem (102, 112) ein auswählbares Drehmerkmal umfasst, um für feste und/oder variable Drehgeschwindigkeiten während der Betätigung des Schneidwerkaufbaus zu sorgen.

26. Eingriffskathetersystem nach einem der Ansprüche 1 bis 25, welches außerdem ein Steuerungssystem (102, 112) zum Bedienen der Antriebswelle und des Schneidwerkaufbaus umfasst, wobei das Steuerungssystem (102, 112) eine Steuerung nur des Motors umfasst, um die Antriebswelle zu aktivieren oder abzuschalten, ohne die Absaugung und/oder die Infusion zu beeinflussen.

27. Eingriffskathetersystem nach einem der Ansprüche 1 bis 26, welches außerdem ein Steuerungssystem (102, 112) zum Bedienen der Antriebswelle und des Schneidwerkaufbaus umfasst, wobei das Steuerungssystem (102, 112) eine Steuerung nur des Absaugens und/oder der Infusion umfasst, um eine Absaug- und/oder Infusionsfunktion zu aktivieren oder abzuschalten, ohne die Antriebswelle zu beeinflussen.

28. Eingriffskathetersystem nach Anspruch 1, welches außerdem ein Steuerungsmerkmal zum Einstellen des Schneidwerkaufbaus mit verstellbaren Klingen (210) zwischen einem Zustand mit größerem Durchmesser und einem Zustand mit kleinerem Durchmesser umfasst.

## Revendications

1. Ensemble de cathéter d'intervention comprenant :
a. un arbre d'entraînement rotatif en communication fonctionnelle avec un système d'entraînement ; et
b. un ensemble d'instrument tranchant (50,72,80,116,120,140) comprenant un ensemble d'instrument tranchant différentiel fixe (50,72,80,120,140,200) comportant une pluralité de surfaces tranchantes différentielles fixes (52,74,77,82,85,124,145,147) dans un agencement radialement symétrique par rapport à un arbre longitudinal central de l'ensemble d'instrument tranchant (50,72,80,116,120,140), dans lequel les surfaces tranchantes différentielles (52,74,77,82,85,124,145,147) ont un angle d'inclinaison inférieur à 90°, l'angle d'inclinaison étant un angle d'intersection de la surface tranchante (52,74,77,82,85,124,145,147) et d'une ligne tangente à la circonférence de l'ensemble d'instrument tranchant (50,72,80,116,120,140) ;
dans lequel l'ensemble d'instrument tranchant (50,72,80,116,120,140) comprend en outre un ensemble d'instrument tranchant à lame réglable (210) comportant une pluralité de lames réglables (150,152,160,162,212) ; et
un système de palier (250) couplant l'ensemble d'instrument tranchant (50,72,80,116,120,140) à l'arbre d'entraînement et à une lumière scellée, le système de palier (250) fournissant une articulation limitée de l'ensemble d'instrument tranchant (50,72,80,116,120,140) par rapport à l'axe de l'arbre d'entraînement, dans lequel le système de palier (250) comprend un arbre interne (260) couplé à l'arbre d'entraînement et un élément statique (280) prévu comme un manchon externe, l'élément statique (280) étant couplé fixement à ou à proximité d'une extrémité proximale vers une extrémité distale du système de cathéter afin de fournir une lumière interne scellée et un système de cathéter.

2. Ensemble de cathéter d'intervention selon la revendication 1, dans lequel des tiges (282) retenues dans des encoches (284) formées sur l'élément statique (280) roulent dans un siège annulaire incurvé (266) formé sur l'arbre interne (260) afin de fournir un pivotement limité de l'arbre interne (260) par rapport à l'élément statique (280).

3. Ensemble de cathéter d'intervention selon la revendication 1 et 12, dans lequel les surfaces tranchantes différentielles fixes (52,74,77,82,85,124,145,147) ont un angle d'inclinaison inférieur à 80°.

4. Ensemble de cathéter d'intervention selon une quelconque des revendications 1 et 3, dans lequel le profil externe de l'ensemble d'instrument tranchant à lame fixe (50,72,80,116,120,140) est généralement ovoïde ou conique quand vu de profil.

5. Ensemble de cathéter d'intervention selon une quelconque des revendications 1 à 4, dans lequel les surfaces tranchantes différentielles fixes (52,74,77,82,85,124,145,147) s'effilent le long d'une ligne incurvée entre une extrémité distale de plus petit diamètre et une extrémité proximale de plus grand diamètre de l'ensemble d'instrument tranchant (50,72,80,116,120,140).

6. Ensemble de cathéter d'intervention selon une quelconque des revendications 1 à 5, comprenant en outre un système d'aspiration incluant une lumière scellée (222) rattachable à une source de vide (104), et dans lequel l'ensemble d'instrument tranchant (50,72,80,116,120,140). Comprend en outre une pluralité de ports (68,125,143) donnant accès à la lumière scellée (222).

7. Ensemble de cathéter d'intervention selon la revendication 6, dans lequel le rapport d'une zone de section transversale totale des ports (68,125,143) comparé à une zone de section transversale totale des surfaces de lame d'instrument tranchant internes est d'au moins 1.5 :1.

8. Ensemble de cathéter d'intervention selon une quelconque des revendications 1 à 7, comprenant en outre un système d'infusion liquide incluant une lumière scellée rattachable à une source d'infusion.

9. Ensemble de cathéter d'intervention selon une quelconque des revendications 1 à 8, dans lequel chaque surface tranchante différentielle (52,74,77,82,85,124,145,147) est prévue sur une surface de lame d'attaque généralement planaire (78,86,131,144).

10. Ensemble de cathéter d'intervention selon une quelconque des revendications 1 à 9, dans lequel chaque surface tranchante différentielle (52,74,77,82,85,124,145,147) est prévue sur un bord terminal externe d'une lame d'instrument tranchant (74,82,122,142) et l'angle formé entre la surface tranchante différentielle (52,74,77,82,85,124,145,147) et une surface de lame de fuite (79,37,130,146) est un angle aigu.

11. Ensemble de cathéter d'intervention selon la revendication 9, dans lequel le bord terminal externe de chaque lame d'instrument tranchant est aiguisé.

12. Ensemble de cathéter d'intervention selon une quelconque des revendications 1 à 11, dans lequel chaque surface tranchante différentielle (52,74,77,82,85,124,145,147) est prévue sur une lame d'instrument tranchant (74,82,122,142), chaque lame d'instrument tranchant (74,82,122,142) aboutit à une extrémité proximale dans un collier proximal (55,128), et chaque surface tranchante différentielle (52,74,77,82,85,124,145,147) a un profil externe qui, à son diamètre le plus grand, est plus grand que le diamètre du collier proximal (55,128).

13. Ensemble de cathéter d'intervention selon une quelconque des revendications 1 à 12, dans lequel l'ensemble d'instrument tranchant à lame réglable (210) est positionné proximalement à l'ensemble d'instrument tranchant différentiel fixe (50,72,80,116,120,140).

14. Ensemble de cathéter d'intervention selon une quelconque des revendications 1 à 13, dans lequel l'ensemble d'instrument tranchant à lame réglable (210) comporte une pluralité de lames d'instrument tranchant agencées radialement (150,152,160,162,212), réglables de manière pivotante entre une orientation tangentielle et une orientation radiale.

15. Ensemble de cathéter d'intervention selon une quelconque des revendications 1 à 14, dans lequel les lames réglables (150,152,160,162,212) ont des surfaces tranchantes différentielles (154,164,214) et les surfaces tranchantes différentielles sur l'ensemble d'instrument tranchant à lames fixes (52,74,77,82,85,124,145,147) et les surfaces tranchantes différentielles sur l'ensemble d'instrument tranchant à lames réglables (154,164,214) ont des angles d'inclinaison différents.

16. Ensemble de cathéter d'intervention selon une quelconque des revendications 1 à 15, dans lequel l'arbre d'entraînement est moins flexible et a une plus grande capacité de résistance au couple à une portion proximale et est plus flexible et a une moins grande capacité de résistance au couple à une portion distale.

17. Ensemble de cathéter d'intervention selon la revendication 16, dans lequel l'arbre d'entraînement est un bobinage hélicoïdal et, à une portion distale de l'arbre d'entraînement, des filetages comprenant le bobinage hélicoïdal sont espacés l'un de l'autre par un espace correspondant à au moins le diamètre d'un filetage.

18. Ensemble de cathéter d'intervention selon une quelconque des revendications 1 à 17, comprenant en outre un élément de scellement installé sur la portion distale de l'arbre d'entraînement, dans lequel l'élément de scellement contacte étroitement mais n'est pas lié à la portion distale de l'arbre d'entraînement.

19. Ensemble de cathéter d'intervention selon la revendication 18, dans lequel l'élément de scellement est formé dans une matière plastique flexible, durable.

20. Ensemble de cathéter d'intervention selon la revendication 19, dans lequel l'élément de scellement est formé en PFTE.

21. Ensemble de cathéter d'intervention selon une quelconque des revendications 1 à 20, comprenant en outre une unité de console (102) en communication avec l'arbre d'entraînement rotatif, dans lequel l'unité de console renferme des fonctions d'alimentation électrique, de commande et d'affichage.

22. Ensemble de cathéter d'intervention selon une quelconque des revendications 1 à 21, comprenant en outre un module de commande (112), dans lequel le module de commande renferme un moteur d'entraînement en communication fonctionnelle avec l'arbre d'entraînement rotatif.

23. Ensemble de cathéter d'intervention selon une quelconque des revendications 1 à 22, dans lequel les surfaces tranchantes différentielles fixes (52,74,77,82,85,124,145,147) ont un angle d'inclinaison inférieur à 70°.

24. Ensemble de cathéter d'intervention selon une quelconque des revendications 1 à 23, comprenant en outre un système de commande (102,112) pour mettre en fonctionnement l'arbre d'entraînement et l'ensemble d'instrument tranchant, dans lequel le système de commande (102,112) comprend une fonctionnalité de couple sélectionnable pour régler le couple rotatif de l'ensemble d'instrument tranchant.

25. Ensemble d'instrument tranchant selon une quelconque des revendications 1 à 24, comprenant en outre un système de commande (102,112) pour mettre en fonctionnement l'arbre d'entraînement et l'ensemble d'instrument tranchant, dans lequel le système de commande (102,112) comprend une fonctionnalité de rotation sélectionnable pour fournir des vitesses de rotation fixes et/ou variables pendant l'actionnement de l'ensemble d'instrument tranchant.

26. Ensemble de cathéter d'intervention selon une quelconque des revendications 1 à 25, comprenant en outre un système de commande (102, 112) pour mettre en fonctionnement l'arbre d'entraînement et l'ensemble d'instrument tranchant, dans lequel le système de commande (102,112) comprend une commande du moteur uniquement afin d'activer ou éteindre l'arbre d'entraînement sans affecter l'aspiration et/ou l'infusion.

27. Ensemble de cathéter d'intervention selon une quelconque des revendications 1 à 26, comprenant en outre un système de commande (102,112) pour mettre en fonctionnement l'arbre d'entraînement et l'ensemble d'instrument tranchant, dans lequel le système de commande (102,112) comprend une commande d'aspiration et/ou d'infusion uniquement afin d'activer ou éteindre une fonction d'aspiration et/ou d'infusion sans affecter l'arbre d'entraînement.

28. Ensemble de cathéter d'intervention selon la revendication 1, comprenant en outre une fonctionnalité de commande pour régler l'ensemble d'instrument tranchant à lames réglables (210) entre une condition de plus grand diamètre et une condition de plus petit diamètre.
